(19) Europäisches Patentamt
European Patent Office
Office européen des brevets

(11) EP 0 563 446 B1

(12) EUROPÄISCHE PATENTSCHRIFT

(45) Veröffentlichungstag und Bekanntmachung des Hinweises auf die Patenterteilung:
14.05.1997 Patentblatt 1997/20

(51) Int. Cl.$^6$: A61K 31/02, A61K 31/08

(21) Anmeldenummer: 92120354.3

(22) Anmeldetag: 27.11.1992

(54) **Behandlungsmittel für die Augenheilkunde und seine Verwendung**

Treating agent for ophthalmology and its use

Agent de traitement pour l'ophthalmologie et son utilisation

(84) Benannte Vertragsstaaten:
AT BE CH DE DK ES FR GB GR IE IT LI LU MC NL PT SE

(30) Priorität: 01.04.1992 DE 4210846
09.04.1992 DE 4211958

(43) Veröffentlichungstag der Anmeldung:
06.10.1993 Patentblatt 1993/40

(73) Patentinhaber: PHARMPUR GmbH
86156 Augsburg (DE)

(72) Erfinder: Meinert, Hasso, Prof. Dr.
W-7910 Neu-Ulm (DE)

(74) Vertreter: Nöth, Heinz, Dipl.-Phys.
Patentanwalt,
Mozartstrasse 17
80336 München (DE)

(56) Entgegenhaltungen:
EP-A- 0 089 232        EP-A- 0 089 815
EP-A- 0 112 658        EP-A- 0 381 986
EP-A- 0 493 677        EP-A- 0 496 299
DE-A- 3 925 525        FR-A- 2 214 674
FR-A- 2 623 525        GB-A-   889 282

• "The Merck Index", 11. Auflage, 1989, Seite 657, Merck & Co., Inc., Rahway, NJ, US
• CHEMICAL ABSTRACTS, Band 83, 1975, Seite 758, Zusammenfassung Nr. 9118r, Columbus, Ohio, US; W. DMOWSKI et al.: "Reactions of sulfur tetrafluoride with carboxylic acids. III. Reactions with alkanecarboxylic, cycloalkanecarboxylic, and certain benzenecarboxylic acids", & ROCZ. CHEM. 1974, 48(10), 1697-706
• J. CHEM. SOC. (C), 1967, Seiten 1450-1454; J.H. ATHERTON et al.: "Reaction of trifluoromethylcarbene with cis- and trans-but-2-enes"
• CHEMICAL ABSTRACTS, Band 90, 1979, Seite 462, Zusammenfassung Nr. 71547c, Columbus, Ohio, US; C. CLEMENT et al.: "Modification of molecular orientation correlations in substituted n-alkanes. Fluoroalkanes", & C.R. HEBD. SEANCES ACAD. SCI., SER. C 1978, 287(6), 227-30
• CHEMICAL ABSTRACTS, Band 87, 1977, Seite 26, Zusammenfassung Nr. 40193t, Columbus, Ohio, US; & JP-A-77 00 887 (DAIKIN KOGYO CO., LTD) 06-01-1977
• CHEMICAL ABSTRACTS, Band 95, 1981, Seite 634, Zusammenfassung Nr. 24384r, Columbus, Ohio, US; & JP-A-81 10 121 (Y. KOBAYASHI) 02-02-1981
• CHEMICAL ABSTRACTS, Band 112, 1990, Seite 700, Zusammenfassung Nr. 98009p, Columbus, Ohio, US; & JP-A-01 180 838 (KANTO DENKA KOGYO CO., LTD) 18-07-1989
• Journal of Fluorine Chemistry (1994), in press
• Journal of Vitreoretina, 1 (2), December 1992, pages 31-35
• Makromol.Chem 189(1988) page 911
• Manuscript of Symposium, pages 20-25, September 1992
• Journal of Fluorine Chemistry 58(1992) page 171
• Tetrahedron 29(1973) pages 2411-2414
• Journal of Fluorine Chemistry vol.4 (1974) pages 261-270
• Biomat.,Art.Cells & Immob.Biotech. vol.19(1991) page 438
• Ibidem vol.20(1991) page 95

# Beschreibung

Die Erfindung betrifft ein Arzneimittel für die Augenheilkunde und seine Verwendung zur Entfaltung der Netzhaut, zur Dauertamponade und als Glaskörperersatz.

Es ist bekannt, daß sich flüssige Perfluorcarbone als Behandlungsflüssigkeit zur Wiederanlegung (Entfaltung) abgelöster Netzhaut an die Aderhaut des Auges eignen (L. Clark, EP 0 089 232; H. Meinert, EP-Anmeldung Nr. 91 12 0184.6).

Weil flüssige Perfluorcarbone eine geeignet hohe Dichte (größer als 1,6 g/cm$^3$) und eine außerordentlich geringe Oberflächenspannung (kleiner als 25 x 10$^{-5}$ N/cm oder 25 dyn/cm) besitzen, eignen sie sich zum Entfalten und Andrücken der abgelösten Netzhaut.

Die geringe Oberflächen- bzw. Grenzflächenspannung verhindert in vorteilhafter Weise ein Hinterlaufen der Behandlungsflüssigkeit hinter die Netzhaut, falls diese Risse aufweist.

Perfluorcarbone sind chemisch und physiologisch inerte Verbindungen, wenn sie hochrein sind.

Die zur Entfaltung abgelöster Netzhaut zu verwendenden Perfluorcarbon-Flüssigkeiten müssen absolut untoxisch sein. Diese Perfluorcarbone dürfen somit keine Verunreinigungen an C-H-Bindungen enthalten, die über eine intramolekulare HF-Abspaltung zur Ausbildung von fluorolefinischen Doppelbindungen führen. Das heißt exakt, daß die einzusetzenden Perfluorcarbon-Flüssigkeiten keine Verunreinigungskomponenten enthalten dürfen, die noch -CHF-Gruppierungen neben -CF$_2$-Gruppierungen in einer Molekel enthalten, weil so eine HF-Abspaltung und damit verbunden eine Toxizität gegeben ist:

$$-CHF-CF_2- \rightarrow -CF=CF- +HF$$

Es ist weiterhin bekannt, daß die zur Entfaltung der Netzhaut verwendeten Perfluorcarbon-Flüssigkeiten nach einer Verweilzeit von Tagen aus dem Auge entfernt und gegen ein anderes Medium ausgetauscht werden.

Dieses Glaskörpersubstitut, wie z.B. Silikonöl, wird für eine längere Tamponade deshalb verwendet, weil die hohe Dichte der Perfluorcarbon-Flüssigkeiten gegen eine Langzeittamponade stehen würde (M. E. Hammer et al. in: H. Mackenzie Freenam, F. I. Tolentino: PVR, Springer-Verlag 1988).

Aufgabe der Erfindung ist es, ein Behandlungsmittel für die Augenheilkunde zu schaffen, das bei der Netzhautbehandlung vielseitig einsetzbar ist.

Die erfindungsgemäße Lösung dieser Aufgabe ergibt sich aus den Patentansprüchen 1 bis 6.

Bevorzugte Ausgestaltungen der Erfindung sind in den Unteransprüchen angegeben.

Wesen der vorliegenden Erfindung ist, als neue Verbindungsklassen modifizierte Fluorkohlenstoffverbindungen der Typen

$$R_F(CH_2)_n R_F$$

$$R_F R_H$$

mit n > 3 und R$_F$ = CF$_3$, C$_2$F$_5$

$$R_H = CH_3(CH_2)_n$$

mit n = 2 bis 10
sowie des Typs

$$R_F(CH_2)_mO(CH_2)_nO(CH_2)_mRF$$

mit R$_F$ = CF$_3$ und CF$_3$(CF$_2$)$_n$
n = 1 bis 4

$$(CH_2)_{n(m)}$$

mit n = 2 bis 6 und
m = 1 bis 4
zu schaffen, die sowohl für die Entfaltung der Netzhaut als auch zur Langzeittamponade und ferner als Glaskörpersubstitut in der Augenheilkunde geeignet sind.

Die modifizierten Fluorkohlenstoffverbindungen oder modifizierten Perfluorcarbone sind chemisch und physiologisch unbedenklich und völlig untoxische farblose Flüssigkeiten, die zur Entfaltung der Netzhaut geeignet sind und ein Hinterlaufen der Netzhaut bei Netzhautrissen ausschließen.

Gegenüber den Perfluorcarbonen, Verbindungen also, die nur aus Kohlenstoff-Fluor-Bindungen bestehen, gegebenenfalls noch ein Heteroatom wie Stickstoff oder Sauerstoff zwischen den die jeweilige Verbindung tragenden CF$_2$-Gruppierungen (kurz als R$_F$-Gruppen bezeichnet) aufweisen, sind die erfindungsgemäß eingesetzten Verbindungen völlig anders aufgebaut. Sie bestehen nämlich aus geschlossenen Kohlenwasserstoff-Alkan-Gruppen, die entweder direkt oder über Sauerstoff-brücken an Perfluoralkylreste gebunden sind.

In diesen vorgeschlagenen Verbindungen kann eine HF-Abspaltung unter Ausbildung fluorolefinischer Doppelbindungen nicht stattfinden, im Gegenteil, die geschlossene Kohlenwasserstoff-Alkan-Gruppierung wirkt noch bindungsverstärkend auf die C-F-Bindungen im Perfluoralkylteil der jeweiligen Verbindung. Sauerstoffatome zwischen den R$_F$- und R$_H$-Gruppierungen schließen ebenfalls eine intramolekulare HF-Abspaltung aus.

Damit sind die erfindungsgemäß verwendbaren Verbindungstypen analog den reinen Perfluorcarbonen chemisch und physiologisch inert und somit untoxisch.

Die erfindungsgemäß verwendbaren perfluorierten Verbindungen oder Verbindungstypen modifizierter Perfluorcarbone besitzen aber einerseits die vorzüglichen Eigenschaften der reinen Perfluorcarbone hinsichtlich der außerordentlich niedrigen Grenzflächenspannung (kleiner als 30 x 10$^{-5}$ N/cm oder 30 dyn/cm), dadurch geprägt, daß am Molekülende R$_F$-Gruppen, d.h. Perfluoralkylgruppen, gebunden sind, so daß sich diese Verbindungen wie die reinen Perfluorcarbone verhalten.

Andererseits werden die Dichten dieser modifizierten Perfluorcarbone sehr wesentlich herabgedrückt auf Werte von 1,2 bis 1,5 $g/cm^3$, dadurch bedingt, daß diese Moleküle einen hohen Anteil an Kohlenwasserstoff-Gruppierungen ($-CH_2-$ bzw. $CH_3-$) und gegebenenfalls auch Sauerstoff-Brückenatome enthalten. Sie besitzen somit wesentlich niedrigere Dichten als die reinen Perfluorcarbone und sind zur Langzeittamponade geeignet.

Diese modifizierten Perfluorcarbone sind somit nicht nur ein Instrument zur Entfaltung der Netzhaut, sondern auch zur Dauertamponade. Für eine Langzeittamponade kann nunmehr ein Austausch der Entfaltungsflüssigkeit (z.B. Perfluorcarbone) gegen eine Flüssigkeit zur Langzeittamponade (z.B. Silikonöl) entfallen.

Die modifizierten Perfluorcarbone sind auch als Glaskörpersubstitut auf Grund ihrer chemischen und physiologischen Inertheit, ihrer relativ zu den Perfluorcarbonen niedrigen Dichte und ihres vorzüglichen Grenzflächenverhaltens sowie auf Grund ihrer sehr guten Löslichkeit für Gase, bevorzugt für Sauerstoff und Kohlendioxid, hervorragend geeignet.

Die modifizierten Perfluorcarbone sind als chemisch sehr stabile Verbindungen mittels geeigneter Reinigungsprozesse als hochreine und somit völlig untoxische Verbindungen darstellbar.

In ihren weiteren physikalischen Eigenschaften verhalten sich diese modifizierten Perfluorcarbone, wie nach dem jeweiligen Molekulargewicht zu erwarten ist, d.h. mit zunehmender Molekülmasse steigen Siedepunkt und Schmelzpunkt an, während der Dampfdruck mit abnehmender Molekülmasse steigt. Damit ergeben sich in vorteilhafter Weise Variationsbreiten für die flüssigen "modifizierten Perfluorcarbone". Ähnlich wie die reinen Perfluorcarbone besitzen die modifizierten Perfluorcarbone eine hohe Löslichkeit für Gase, u.a. für $O_2$ und $CO_2$. Die verwendbaren modifizierten Perfluorcarbone sind analog den Perfluorcarbonen und den Kohlenwasserstoffen in Wasser kaum bis nicht löslich.

Die für die Augenheilkunde interessanten flüssigen modifizierten Perfluorcarbone sind farblose Flüssigkeiten, die auf Grund ihrer hervorragenden chemischen Resistenz sich auch bei einer Laserbehandlung (Laserkoagulation) vorzüglich eignen, weil keine Zersetzungsprodukte bei der Laserbestrahlung entstehen.

Die erfindungsgemäß verwendbaren Verbindungen lassen sich für den Einsatz in der Augenheilkunde hochgereinigt darstellen.

Dazu werden die modifizierten Perfluorcarbone zunächst mit saurer Permanganatlösung gekocht. Danach werden sie mit einem Gemisch von starker wäßriger Kalilauge (4 bis 8 n), CaO bzw. BaO, und einem nukleophilen Agenz (sekundäres Amin und/oder einwertiger Alkohol) bei 150 - 180°C ca. 72 Stunden lang autoklaviert. Das autoklavierte Produkt wird abschließend mittels Scheidetrichter von der wäßrigen alkalischen Phase, die gegebenenfalls noch Alkohol enthält, und der gegebenenfalls vorhandenen Aminphase abgetrennt, mehrmals nacheinander mit verdünnter Mineralsäure, $NaHCO_3$-Lösung, destilliertem Wasser, wasserfreien $Na_2SO_4$ und wasserfreiem $CaCl_2$ behandelt und zum Schluß über eine leistungsfähige Kolonne fraktioniert destilliert.

**Patentansprüche**

1. Verwendung flüssiger modifizierter Perfluorkohlenwasserstoffe des Typs

$$R_F(CH_2)_n R_F$$

$$R_F R_H$$

mit $n > 3$ und $R_F = CF_3, C_2F_5$

$$R_H = CH_3(CH_2)_n$$

mit $n = 2$ bis $10$
oder des Typs

$$R_F(CH_2)_m O(CH_2)_n O(CH_2)_m RF$$

mit $R_F = CF_3$ und $CF_3(CF_2)_n$
$n = 1$ bis $4$

$$(CH_2)_{n(m)}$$

mit $n = 2$ bis $6$ und
$m = 1$ bis $4$
zur Herstellung eines Arzneimittels für die Augenheilkunde.

2. Verwendung nach Anspruch 1 zur Herstellung eines Mittels zur Netzhautentfaltung.

3. Verwendung nach Anspruch 1 zur Herstellung eines Tamponademittels für eine wieder angelegte Netzhaut.

4. Verwendung nach Anspruch 1 zur Herstellung eines Augenglaskörperersatzes.

5. Verwendung nach Anspruch 1 bei der Laserkoagulation.

6. Verwendung nach einem der Ansprüche 1 bis 5, wobei das modifizierte Perfluorcarbon eine Dichte von 1,2 bis 1,5 $g/cm^3$ aufweist.

**Claims**

1. Use of liquid modified perfluorohydrocarbons of the type

$$R_F(CH_2)_n R_F$$

$$R_FR_H$$

with n > 3 and $R_F$ = $CF_3$, $C_2F_5$

$$R_H = C_{H3}(CH_2)_n$$

with n = 2 to 10
or the type

$$R_F(CH_2)_mO(CH_2)_nO(CH_2)_mR_F$$

with $R_F$ = $CF_3$ and $CF_3(CF_2)_n$
n = 1 to 4

$$(CH_2)_{n(m)}$$

with n = 2 to 6 and
m = 1 to 4
for the production of a medication for ophthalmology.

2. Use according to claim 1 for the production of an agent for unfolding a retina.

3. Use according to claim 1 for the production of a tamponade agent for a reapplied retina.

4. Use according to claim 1 for the production of a vitreous humor substitute.

5. Use according to claim 1 in laser coagulation.

6. Use according to one of claims 1 to 5 wherein the modified perfluorocarbon has a density of 1.2 to 1.5 g/cm$^3$.

**Revendications**

1. Utilisation d'hydrocarbures perfluorés liquides modifiés du type,

$$R_F(CH_2)_n R_F$$

$$R_FR_H$$

avec n > 3 et $R_F$ = $CF_3$, $C_2F_5$

$$R_H = CH_3 (CH_2)_n$$

avec n = 2 à 10
ou du type

$$R_F(CH_2)_mO(CH_2)_nO(CH_2)_mRF$$

avec $R_F$ = $CF_3$ et $CF_3 (CF_2)_n$
n = 1 à 4

$$(CH_2)_{n(m)}$$

avec n = 2 à 6 et
m = 1 à 4
pour la préparation d'un médicament pour l'ophtalmologie.

2. Utilisation suivant la revendication 1 pour la préparation d'un agent pour le déplissage de la rétine.

3. Utilisation suivant la revendication 1, pour la préparation d'un agent de tamponnade pour une rétine réappliquée.

4. Utilisation suivant la revendication 1, pour la préparation d'un ersatz du vitré oculaire.

5. Utilisation suivant la revendication 1, pour une coagulation au laser.

6. Utilisation suivant l'une des revendications 1 à 5, le perfluorocarbone modifié présentant une masse volumique de 1,2 à 1,5 g/cm$^3$.